# EUROPEAN PATENT SPECIFICATION

(11) **EP 0 357 351 B1**
(45) Date of publication and mention of the grant of the patent: **20.10.1993**
(21) Application number: 89308649.6
(22) Date of filing: 25.08.1989
(51) Int. Cl.: A61K 7/06

(54) **Treatment compositions**
Behandlungszusammensetzungen
Compositions de traitement

(30) Priority: 02.09.1988 US 240787
(43) Date of publication of application: 07.03.1990
(73) Proprietor: Bristol-Myers Squibb Company, New York, N.Y. 10154 (US)
(72) Inventor: DeMarco, Richard, Danbury Connecticut 06810 (US); Feinland, Raymond, Stamford Connecticut 06905 (US); Jachowicz, Janusz, Bethel Connecticut 06801 (US)
(74) Representative: Baillie, Iain Cameron

(56) References cited:
- EP-A- 0 047 714
- DE-A- 3 347 189
- GB-A- 2 071 495
- GB-A- 2 096 180
- GB-A- 2 114 580

## Description

Chemical processing such as the coloring, straightening and permanent waving of human hair and other proteinaceous substrates can be damaging to those substrates. It is known in the art to protect or, in some respects, "stabilize" the hair, before, during or after such treatment, with a composition containing protective agents.

U.S. 4,371,517, to Vanlerberghe et al, describes the use of a combination of a cationic polymer, an anionic polymer, an alkali metal salt, and a non-ionic surfactant which contains carboxyl or carboxylated groups in compositions to be used to wash or dye hair. Copolymers containing tetraalkyl ammonium halide units are disclosed at column 4.

U.S. 4,645,663 to Grollier et al deals with hair dyeing or bleaching compositions which may contain copolymers having tetralklyammonium halide units (see column 9).

Both of these patents refer to treatment systems in which maximum conditioning effectiveness requires the conjoint use of either an anionic polymeric component or an anionic surfactant with the cationic copolymers.

U.S. patent 3,912,808 and 3,986,825 to Sokol disclose similar systems of cationic copolymers in which surfactant is either present in the compositions or used in post-treatments.

### The Invention

The invention deals with compositions and processes for conditioning hair or other proteinaceous substrates.

Applicants have discovered that the treating of hair or other proteinaceous substrates with compositions containing a copolymer of about 10% hexadecyldimethylpropyl methacrylamide ammonium bromide (HDPM) with about 90% methylacrylamidopropyltrimethyl ammonium chloride (MAPTAC), which copolymers are referred to as "H-QUAT" polymers, with other chemical treating materials, e.g., coloring, bleaching, perming and/or shampooing compositions, made the hair more easily combable and manageable.

### Advantages

The compositions of the invention have several advantages over known compositions and processes for treating hair and other fibrous substrates to render them more manageable and to stabilize them against the harsh side effects of chemical processing.

One major advantage resides in the fact that compositions containing cationic H-QUAT polymers need not be used in conjunction with anionics in order to effect beneficial results. Thus, unlike other systems described above, the cationics can be used in one-step conditioning as the sole polymeric agents in the protective compositions and processes of the invention.

Note that many cationic/anionic combinations result in "over conditioning" problems when the polymer precipitates into the hair. Such problems are minimized using the instant system. In fact, the "over conditioning" which is typical with the use of cationic/anionic complexes or combinations is virtually eliminated using the instant invention because no anionic polymer or surfactant complexes or combinations are needed for the operability of the invention.

In addition, the high miscibility of these cationic polymers with aqueous solvents and other polar liquids make their formulation both easy and inexpensive.

Furthermore, the processes of the invention produce improvements in the treated hair, e.g. less tangling and static and better feel. These improvements are durable, generally lasting through several, e.g. 2-5, shampoos.

These and other aspects and advantages of the invention will become more apparent after consideration of the following description of the invention.

### Description of the Invention

The invention is concerned with processes involving the use of specified polymeric compositions for treating proteinaceous or other fibrous substrates.

Copolymers employed in the specified polymeric compositions are cationic copolymers which contain from 1 to 20 mole %, preferably 5 to 15 mole %, most preferably about 10 mole % of units of hexadecyldimethylpropylmethacrylamide ammonium halide and 80 to 99 moles %, preferably 85 to 95%, most preferably about 90 mole % of units of methacrylamidopropyltrimethyl ammonium halide. One particularly preferred polymer of this type is the 'H-Quat' polymer which contains 10% hexadecyldimethylpropylmethacrylamide ammonium bromide (HDPM) and 90% methacrylamidopropyltrimethyl ammonium chloride.

While units of the above mentioned formulae are essential in the cationic species to be used, the presence of other vinyl residues is contemplated. Thus, useful copolymers may contain moieties derived from one or more other unsaturated species, such as ethylene, propylene, C₁₋₆ (meth)acrylates, and C₁₋₆ (meth)acrylamides.

These polymeric component(s) may be used in combination with other polymers. However, no other polymers need be present. The polymeric components of applicants' composition are cationic polymers which requires no additional anionic or nonionic species for "balance". While applicants' copolymers are within the broad grouping of polymers disclosed in U.S. patents 4,371,517 and 4,645,663 the sole use of the specific polymers described above for applicants' purposes is not taught therein.

Although anionic surfactants are not required for effect in the product, their use is not precluded.

The compositions of the invention are generally applied from a liquid or spray formulations. Optionally, they may be formulated into gels, creams, or other forms.

The compositions of the invention contain one or more diluents or carriers. Typically, the carrier employed will be aqueous, e.g. water alone, or water/C₁₋₃ alcohol combinations, or C₁₋₃ alcohols alone.

Applicants' treatment compositions contain from 0.05 to 1 wt.%, preferably 0.2 to 0.5 wt.%, of one or more polymeric additive(s), with the remainder being one or more carrier(s) or other conventional additives.

Conventional excipients or additives may be used in suitable quantities in the compositions of the invention. Thus, depending upon the overall function of the treating composition, one or more surfactants, plasticizers, soaps, stabilizers, fillers, thixotropic agents, colorants, perfumes, buffers and the like may be employed.

When the polymeric components of the invention are used in post-shampoo rinses, e.g. in conditioning formulations, they will be present at concentration levels of 0.05 to 5 wt.% preferably 1 to 1.5wt.%.

### Hair Treatment

The compositions of the invention can be applied to proteinaceous substrates of a variety of types. While the treatment of hair on the human head is highly preferred, the compositions can also be used to treat hair which is not on a body, e.g. in wigs, swatches, and garments made of hair. It may also be applied to non-human hair or fur, e.g., wool or dog hair as well as pelts or skins of other animals.

Alternatively, the compositions can be used to treat keratin fibers such as hair and wool. Blends, e.g., polymer/wool, can also be present in substrates to be treated.

These compositions are usually applied in any suitable liquid form such as sprays, lotions or creams. Thus, they can be formulated using the carrier(s) and/or other excipients discussed above in order to achieve requisite properties, e.g. viscosity, stability and handling properties.

While liquid formulations are preferred, the use of semi-solids and solids is contemplated. Thus, gels, mousses, creams as well as hot melt formulations are envisioned.

When a liquid formulation is sprayed onto the hair it is visually used in the form of a solution of the polymeric component in a carrier such as water or mixtures of water and ethanol.

In the preferred embodiment, the composition is applied one or more times via spraying from a conventional non-aerosol spray device onto the hair or other substrate to deposit .01 to .02g. of polymer on a typical head of hair. The chemical treatment is then applied without attempting to rinse the hair. Shampooing may be done after the chemical treatment is used.

While it is preferred that the compositions be applied before any chemical treatment, i.e., as pre-treatment compositions, they may also be used during, and/or after the use of conventional chemical agents.

### Examples

The following examples are given to further illustrate the present invention. Compositions were prepared comparing H-Quat, poly(methacrylamidopropyltrimethyl ammonium chloride (PMAPTAC), polyquaternium 10 and polyquaternium 6 as the actives and applied to hair previously tinted and therefore usually difficult to comb and hard to manage. The compositions were applied in the form of a spray.

### Examples I

Hair Tint Composition I was mixed with 20 volume hydrogen peroxide and applied immediately thereafter. It was allowed to remain on hair swatches of 2 g. size for 30 minutes and rinsed off. Two controls were not pre-treated. One of these controls was post-treated with a Standard Conditioner Composition (Composition E) which is the usual method for conditioning hair.

| HAIR TINT COMPOSITION I | PERCENT |
|---|---|
| P-Phenylenediamine | 2.20 |
| Resorcinol | 1.10 |
| M-aminophenol | 1.00 |
| Sodium Sulfite | 0.10 |
| EDTA | 0.05 |
| Propylene Glycol | 5.00 |
| Isopropanol | 10.00 |
| Nonoxynol-4 | 3.50 |
| Cetyl Alcohol | 5.00 |
| Ammonium Hydroxide | 9.00 |
| Ceteareth-20 | 3.50 |
| Water | 59.45 |
| TOTAL | 100.00 |

The following conditioning compositions were used to pre-treat the hair swatches:

| Composition A | Percent |
|---|---|
| H-Quat | 0.24 |
| Water | 99.76 |

| Composition B | Percent |
|---|---|
| POLYMAPTAC | 0.24 |
| Water | 99.76 |

| Composition C | Percent |
|---|---|
| Polyquaternium 10 | 0.24 |
| Water | 99.76 |

| Composition D | Percent |
|---|---|
| Polyquaternium 6 | 0.24 |
| Water | 99.76 |

| Standard Conditioner Composition | |
|---|---|
| (Composition E) | Percent |
| Ceteareth-20 | 3.5 |
| Cethyl Alcohol | 1.0 |
| Strealkonium Chloride | 2.0 |
| Citric Acid | 3.0 |
| Water | 90.5 |
| | 100.0 |

The swatches were rinsed and then evaluated for ease of wet combing before shampooing and after lathering with shampoo. A scale was used with the untreated control assigned zero and the control post treated with standard conditioner composition E assigned 5.

Table I clearly illustrates the efficacy and durability of H-Quat.

**TABLE I**

| Combability of tinted hair (1) immediately after treatment with conditioner and (2) after three latherings with shampoo*. | | |
|---|---|---|
| Composition | Before Shampooing | After 3 Latherings |
| A. H-Quat | 4.0 | 4.0 |
| B. POLYMAPTAC | 1.5 | 0.0 |
| C. Polyquaternium 10 | 0.0 | 0.0 |
| D. Polyquaternium 6 | 1.5 | 0.0 |

| E. Standard Conditioner | | |
|---|---|---|
| Composition | 5.0 | 0.0 |
| Control | 0.0 | 0.0 |

| | | |
|---|---|---|
| * The shampoo used was condition* Shampoo from Clairol. | | |

### Example II

Previously tinted hair was permed with the following composition:

| PERM COMPOSITION FOR TINTED HAIR | PERCENT |
|---|---|
| Ammonium Thioglycolate (60% as acid) | 12.2 |
| Laureth-23 | 1.5 |
| Fragrance | 1.0 |
| EDTA | 0.1 |
| Ammonium Hydroxide | 3.5 |
| Water | 81.7 |
| TOTAL | 100.0 |
| The perm composition was neutralized with 2.0% Hydrogen Peroxide. | |

Prior to perming, swatches of the size used above were treated with the compositions of A-D in the same manner. Two control swatches were made without a pre-treatment. One of the controls was post-treated with Standard Conditioner Composition E. The swatches were then evaluated for ease of wet combing after neutralization of the perm and after three lathers with Condition* Shampoo by Clairol.

A combability scale was assigned with the untreated control assigned zero and the control post treated with Standard Conditioner Composition was assigned 5. Table II clearly illustrates the results.

**TABLE II**

| Combability of tinted permed hair (1) immediately after treatment with conditioner and (2) after three latherings with shampoo | | |
|---|---|---|
| Composition | Before Shampooing | After 3 Latherings |
| A. H-Quat (Example I) | 4.5 | 4.5 |
| B. Polympatac (Example II) | 2.5 | 0.0 |
| C. Polyquaternium 10 (Example III) | 0.0 | 0.0 |
| D. Polyquaternium 6 (Example IV) | 3.0 | 1.0 |

| E. Standard Conditioner | | |
|---|---|---|
| Composition | 5.0 | 0.0 |
| Control | 0.0 | 0.0 |

Conditioning occurs during the chemical treatment and persists after the hair is washed and styled.

### Example III

To further illustrate the invention, the compositions A-D were applied prior to the application of A Standard Hair Straightening Composition. The straightener was left on ten minutes and then rinsed.

| STANDARD HAIR STRAIGHTENER | PERCENT |
|---|---|
| Water | 74.25 |
| Propylene Glycol | 2.00 |
| Mineral Oil | 10.00 |
| Emulsifying Wax NF | 5.00 |
| Laneth-15 | 1.00 |
| Cetyl Alcohol | 1.00 |
| Ceteareth-20 | 3.00 |
| Laneth 5 and Ceteth-25 | 2.00 |
| Sodium Hydroxide | 1.75 |
| TOTAL | 100.00 |

Two control swatches of the same size as those used above were made. One swatch was only treated with Standard Hair straightener and the other was post-treated with Standard Conditioning Composition E. The swatches were evaluated for ease of wet combing. A scale of 0 to 5 was used with the control assigned 0 and the swatch post treated with Standard Conditioning Composition assigned 5. The results are set out in Table III.

**TABLE III**

| Combability of Hair Post-Straightened after treatment with conditioner with combability measured, before and after shampooing | | |
|---|---|---|
| Composition | Before Shampooing | After 3 Latherings |
| A. H-Quat | 5.0 | 4.0 |
| B. POLYMAPTAC | 3.5 | 2.0 |
| C. Polyquaternium 10 | 1.0 | 0.0 |
| D. Polyquaternium 6 | 3.5 | 2.0 |

| E. Standard Conditioner | | |
|---|---|---|
| Composition | 5.0 | 0.0 |
| Control | 0.0 | 0.0 |

The foregoing examples have illustrated the ability of H-Quat to condition without the necessity of complexing with an anionic surfactant and its improved durability by surviving various processing steps and subsequent shampooings.

## Claims

1. A method of treating a proteinaceous substrate to render it more manageable comprising the step of applying to the substrate a composition comprising:
(a) water, and
(b) a cationic copolymer which contains, on a mole basis, 1% to 20% hexadecyldimethylpropylmethacrylamide ammonium halide and 80% to 99% methacrylamidopropyltrimethyl ammonium halide, wherein the halide is selected from chloride and bromide.

2. A method of conditioning and coloring a proteinaceous substrate comprising the steps of:
(a) pretreating the substrate with a conditioning composition comprising a cationic polymer which contains, on a mole basis, 1% to 20% of hexadecyldimethylpropylmethacrylamide and 80% to 99% methacrylamidopropyltrimethyl ammonium halide wherein the halide is selected from chloride and bromide; and
(b) applying a hair coloring composition to the substrate, wherein the pretreatment step (a) improves wet combability.

3. The method of any preceding claim wherein the substrate is human hair.

4. The method of any preceding claim wherein the cationic polymer contains, on a mole basis, about 10% hexadecyldimethylpropylmethacrylamide ammonium bromide and about 90% methacrylamidopropyltrimethyl ammonium chloride.

## Patentansprüche

1. Verfahren zur Behandlung eines Proteinsubstrats, damit es besser zu handhaben ist, wobei bei diesem Verfahren eine Zusammensetzung auf das Substrat aufgebracht wird, die folgendes enthält:
(a) Wasser, und
(b) ein kationisches Copolymer, das auf Molbasis 1% bis 20% Hexadecyldimethylpropylmethacrylamid-Ammoniumhalogenid und 80% bis 90% Methacrylamidopropyltrimethyl-Ammoniumhalogenid enthält, wobei das Halogenid ausgewählt ist aus Chlorid und Bromid.

2. Verfahren zum Vorbehandeln und Färben eines Proteinsubstrats, umfassend die folgenden Schritte:
(a) Vorbehandeln des Substrats mit einer Vorbehandlungszusammensetzung, die ein kationisches Polymer enthält, welches auf Molbasis 1% bis 20% Hexadecyldimethylpropylmethacrylamid und 80% bis 99% Methacrylamidopropyltrimethyl-Ammoniumhalogenid enthält, wobei das Halogenid ausgewählt ist aus Chlorid und Bromid; und
(b) Auftragen einer Haarfärbemischung auf das Substrat, wobei der Vorbehandlungsschritt (a) die Kämmbarkeit im nassen Zustand verbessert.

3. Verfahren nach einem der vorhergehenden Ansprüche, dadurch gekennzeichnet, daß das Substrat menschliches Haar ist.

4. Verfahren nach einem der vorhergehenden Ansprüche, dadurch gekennzeichnet, daß das kationische Polymer auf Molbasis etwa 10% Hexadecyldimethylpropylmethacrylamid-Ammoniumbromid und etwa 90% Methacrylamidopropyltrimethyl-Ammoniumchlorid enthält.

## Revendications

1. Procédé de traitement d'un substrat protéinique pour le rendre plus pratique, ledit procédé comprenant l'étape d'application au substrat d'une composition renfermant :
(a) de l'eau, et
(b) un copolymère cationique qui contient, en pourcentage molaire, 1 à 20 % d'halogènure d'hexadécyldiméthylpropylméthacrylamide-ammonium et 80 à 99 % d'halogènure de méthacrylamidopropyltriméthylammonium, où l'halogènure est choisi parmi le chlorure et le bromure.

2. Procédé de conditionnement et de coloration d'un substrat protéinique comprenant les étapes consisantes à :
(a) prétraiter le substrat avec une composition de conditionnement comprenant un polymère cationique qui contient, en pourcentage molaire, 1 à 20 % d'halogènure d'hexadécyldiméthylpropylméthacrylamide-ammonium et 80 à 99 % d'halogènure de méthacrylamidopropyltriméthylammonium, où l'halogènure est choisi parmi le chlorure et le bromure ; et
(b) appliquer la composition de coloration pour cheveux au substrat, le prétraitement de l'étape (a) améliorant le peignage à l'état humide.

3. Procédé suivant l'une quelconque des revendications précédentes, dans lequel le substrat est le cheveu humain.

4. Procédé suivant l'une quelconque des revendications précédentes, dans lequel le polymère cationique contient, en pourcentage molaire, environ 10 % de bromure d'hexadécyldiméthylpropylméthacrylamide-ammonium et 90 % de chlorure de méthacrylamidopropyltriméthylammonium.
